# EUROPEAN PATENT APPLICATION

(11) **EP 2 977 763 A1**
(43) Date of publication of application: **27.01.2016**
(21) Application number: 14769141.4
(22) Date of filing: 20.03.2014
(51) Int. Cl.: G01N 33/68, C07K 16/18, C12N 15/09, C12Q 1/68, G01N 27/62, G01N 33/50, G01N 33/53, G01N 33/574

(54) **ANALYSIS METHOD FOR ASSESSING STAGE OF PROSTATE CANCER, PROSTATE-CANCER STAGE ASSESSMENT METHOD, PROSTATE-CANCER DETECTION METHOD, AND TEST KIT**

(30) Priority: 22.03.2013 JP 2013061094
(71) Applicant: Riken, Wako-shi, Saitama 351-0198 (JP)
(72) Inventor: UEDA, Koji, Wako-shi Saitama 351-0198 (JP); NAKAGAWA, Hidewaki, Wako-shi Saitama 351-0198 (JP)
(74) Representative: Graf von Stosch, Andreas
(86) International application number: PCT/JP2014/057879
(87) International publication number: WO 2014/148627

(57) **Abstract**

Each of (i) an analysis method of the present invention for assessing a degree of progression of prostate cancer and (ii) a method of the present invention for assessing a degree of progression of prostate cancer includes the step of measuring an amount of neuropeptide Y in a sample derived from blood or urine obtained from a living organism. A method of the present invention for detecting prostate cancer includes the step of measuring respective amounts of neuropeptide Y and prostate-specific antigen in a sample derived from blood or urine obtained from a living organism.

## Description

### Technical Field

The present invention relates to an analysis method for assessing a stage (a degree of progression) of prostate cancer, a prostate-cancer stage assessment method (a method for assessing a degree of progression of prostate cancer), a prostate-cancer detection method (a method for detecting prostate cancer), a test kit for assessing a stage of prostate cancer, and a test kit for detecting prostate cancer.

### Background Art

Prostate-specific antigen (PSA), which is also known as kallikrein-3, was discovered in 1969 (Non-Patent Literature 1), and had been recognized as a best diagnosis tool for prostate cancer since the Food and Drug Administration (FDA) approved the PSA test in 1994 (Non-Patent Literature 2). Indeed, at least 70% of 44 million men aged 50 or older undergo the PSA test in the U.S. in a year. However, in October 2011, the U.S. Preventive Services Task Force (USPSTF) published statistical evidences about the clinical outcomes of prostate cancer and urged not to continue the PSA test on healthy men any more (Non-Patent Literature 3). This decision is simply based on the concept that the benefit of the PSA test with respect to an overall survival rate of prostate cancer patients does not worth the risk of expanding invasive prostate biopsy cases and medical costs. It was estimated that 5.2 million U.S. dollars would be spent for PSA screening in order to prevent one death due to prostate cancer (Non-Patent Literature 4).

So far, prostate cancer biomarker candidates other than PSA have been searched. For example, Non-Patent Literature 5 states that, according to the study in which prostate tissues are collected from patients with prostate diseases and are stained by immunostaining, neuropeptide Y (NPY) and macrophage inhibitory cytokine-1 (MIC-1) are highly expressed in prostate tissues of prostatic intraepithelial neoplasia (PIN) patients and prostate cancer patients. Further, Patent Literature 1 states that an amount of mRNA encoding NPY is greater in prostate tumor tissues than in normal prostate tissues.

### Citation List

### [Patent Literature]

[Patent Literature 1]
   Japanese Patent Application Publication (Translation of PCT Application), *Tokuhyo,* No. 2005-523727 (Publication Date: August 11, 2005)

### [Non-Patent Literature]

[Non-Patent Literature 1]
   Ablin, R. J., Pfeiffer, L., Gonder, M. J., and Soanes, W. A. (1969), Experimental medicine and surgery 27, 406-410.
[Non-Patent Literature 2]
   Catalona, W. J., Smith, D. S., Ratliff, T. L., Dodds, K. M., Coplen, D. E., Yuan, J. J., Petros, J. A., and Andriole, G. L. (1991), The New England journal of medicine 324, 1156-1161.
[Non-Patent Literature 3]
   Chou, R., Croswell, J. M., Dana, T., Bougatsos, C., Blazina, I., Fu, R., Gleitsmann, K., Koenig, H. C., Lam, C., Maltz, A., Rugge, J. B., and Lin, K. (2011), Annals of internal medicine 155, 762-771.
[Non-Patent Literature 4]
   Brett, A. S., and Ablin, R. J. (2011), The New England journal of medicine 365, 1949-1951.
[Non-Patent Literature 5]
   Krishan K. Rasiah, James G. Kench, Margaret Gardiner-Garden, Andrew V. Biankin, David Golovsky, Phillip C. Brenner, Raji Kooner, Gordon F. O' Neill, Jennifer J. Turner, Warick Delprado, C. Soon Lee, David A. Brown, Samuel N. Breit, John J. Grygiel, Lisa G. Horvath, Phillip D. Stricker, Robert L. Sutherland, and Susan M. Henshall, (2006), Cancer Epidemiol Biomarkers Prev 2006; 15(4), 711-716.

### Summary of Invention

### Technical Problem

As described above, PSA has been used as a prostate cancer biomarker in a clinical site. However, in order to reduce the above-stated risk derived from overdiagnosis of prostate cancer, there is a strong demand for development of a detection method which is able to effectively complement a poor specificity of PSA.

In order to assess a degree of progression of prostate cancer, typically, there is no way but to conduct a highly invasive biopsy and then to make an objective judgment based on a pathologic image obtained by a microscope. If it is possible to assess a degree of progression of prostate cancer at an early stage, e.g., at the stage of blood test, this will significantly reduce the burden on a patient and allow prompt decision of a therapeutic strategy for the patient based on the subjective criteria. Thus, there is also a demand for development of a method for assessing a degree of progression of prostate cancer which method can be carried out at an early stage.

Non-Patent Literature 5 describes the expression amount of NPY in the prostate tissues. However, Non-Patent Literature 5 does not indicate an existing amount of NPY in other samples (e.g., blood or urine). With regard to various proteins, the fact is known that there is no correlation between (i) an expression amount of a protein in tissues and (ii) an existing amount of the protein in samples derived from others. Furthermore, Non-Patent Literature 5 indicates that an expression amount of NPY in prostate tissues has no correlation with a degree of progression of prostate cancer. In addition, Patent Literature 1 does not describe a relationship between an amount of NPY peptide and a degree of progression of prostate cancer. Moreover, none of Non-Patent Literature 5 and Patent Literature 1 indicates experimental data for sensitivity and specificity.

The present invention was made in view of the above problems. An object of the present invention is to provide an analysis method for assessing a degree of progression of prostate cancer, a method for assessing a degree of progression of prostate cancer, a method for detecting prostate cancer which method is able to effectively complement a poor specificity of PSA.

### Solution to Problem

An analysis method according to the present invention for assessing a degree of progression of prostate cancer includes the step of measuring an amount of neuropeptide Y in a sample derived from blood or urine obtained from a living organism.

A method according to the present invention for detecting prostate cancer includes the step of measuring respective amounts of neuropeptide Y and prostate-specific antigen in a sample derived from blood or urine obtained from a living organism.

A method according to the present invention for assessing a degree of progression of prostate cancer includes the step of measuring an amount of neuropeptide Y in a sample derived from blood or urine obtained from a living organism.

A test kit according to one aspect of the present invention is a test kit for assessing a degree of progression of prostate cancer, the test kit including a peptide probe for measuring an amount of neuropeptide Y in a sample derived from blood or urine obtained from a living organism.

A test kit according to another aspect of the present invention is a test kit for assessing a degree of progression of prostate cancer, the test kit including an antibody for measuring an amount of neuropeptide Y in a sample derived from blood or urine obtained from a living organism, and the test kit further including at least one of (i) an antibody or a peptide probe each for measuring an amount of prostate-specific antigen in a sample derived from blood or urine obtained from the living organism, (ii) a nucleic-acid probe or a nucleic-acid primer each for measuring an amount of mRNA of TMPRSS2:ERG in a sample derived from blood or urine obtained from the living organism, and (iii) a nucleic-acid probe or a nucleic-acid primer each for measuring an amount of PCA3 RNA in a sample derived from blood or urine obtained from the living organism.

A test kit according to further another aspect of the present invention is a test kit for detecting prostate cancer, the test kit including: an antibody or a peptide probe each for measuring an amount of neuropeptide Y in a sample derived from blood or urine obtained from a living organism; and an antibody or a peptide probe each for measuring an amount of prostate-specific antigen in a sample derived from blood or urine obtained from the living organism.

The present specification includes the contents of the specification and/or the drawings of the Japanese Patent Application No. 2013-061094, based on which the present application claims priority.

### Advantageous Effects of Invention

The present invention is able to provide an analysis method for assessing a degree of progression of prostate cancer, a method for assessing a degree of progression of prostate cancer, and a method for detecting prostate cancer which is able to effectively complement a poor specificity of PSA.

### Brief Description of Drawings

Fig. 1 shows an overall workflow of RefinerMS software in an Example.
Fig. 2 shows (i) an outline of QUEST-MS technology in the Example and (ii) results of purification carried out by the QUEST-MS technology in the Example.
Fig. 3 shows results of screening for a prostate cancer biomarker by an absent-present search in Expressionist proteome server in the Example.
Fig. 4 shows results of immunohistochemical staining analysis of NPY in the Example.
Fig. 5 shows optimization of MRM conditions for NPY that were applied to a validation experiment for a biomarker in the Example.
Fig. 6 shows evaluation of quantitative reproducibility of MRM-based NPY measurement in the Example.
Fig. 7 is a view for comparison between ROC curves generated for NPY (an area of MRM chromatogram), PSA (values measured by ELISA in hospitals), and NPY + PSA (logistic regression scores), respectively, in the Example.
Fig. 8 is a view for evaluating a diagnostic potential by the ROC curves in the Example.
Fig. 9 shows plasma NPY levels in samples collected before and after a surgical operation in the Example.

### Description of Embodiments

For the present invention, the inventors of the present invention uniquely developed the QUEST-MS (Quick Enrichment of Small Targets for Mass Spectrometry) technology, which is useful in searching for a biomarker. Further, the inventors of the present invention employed the QUEST-MS technology in order to identify a prostate cancer biomarker. As a result, the inventors of the present invention found that (i) neuropeptide Y (NPY) is highly relevant to a degree of progression of prostate cancer and (ii) NPY is able to effectively complement a poor specificity of PSA. Thus, the inventors of the present invention completed the present invention.

### [1. An analysis method for assessing a degree of progression of prostate cancer and a method for assessing a degree of progression of prostate cancer]

An analysis method according to the present invention for assessing a degree of progression of prostate cancer includes the step of measuring an amount of neuropeptide Y (NPY) in a sample derived from blood or urine obtained from a living organism.

A method according to the present invention for assessing a degree of progression of prostate cancer includes the step of measuring an amount of NPY in a sample derived from blood or urine obtained from a living organism.

Each of the analysis method and the assessment method according to the present invention preferably includes, in addition to the step of measuring the amount of NPY, the step of measuring an amount of at least one of prostate-specific antigen (PSA), mRNA of TMPRSS2:ERG, and PCA3 (prostate cancer antigen 3; prostate cancer gene 3) RNA. Each of the analysis method and the assessment method according to the present invention further preferably includes, in addition to the step of measuring the amount of NPY, the step of measuring an amount of PSA.

Each of the polypeptides (NPY and PSA) that can be measured in the present invention is hereinafter referred to as a "prostate cancer marker peptide". Further, each of RNAs (mRNA of TMPRSS2:ERG and PCA3 RNA) that can be measured in the present invention is hereinafter referred to as "prostate cancer marker RNA". Furthermore, each of the "prostate cancer marker peptide" and the "prostate cancer marker RNA" is hereinafter referred to as a "prostate cancer marker".

The term "prostate cancer marker peptide" encompasses not only a full-length of the polypeptide described above but also a fragment of the polypeptide and a precursor of the polypeptide. Namely, for example, the expression "measuring an amount of NPY" means measuring at least one of a full-length NPY polypeptide, a fragment of NPY polypeptide, and a precursor of NPY polypeptide. NPY whose amount is to be measured can be, for example, NPY₁₋₉₇, NPY₁₋₂₈, NPY₂₉₋₉₇, NPY₆₈₋₉₇, NPY₂₉₋₆₇, or NPY₂₉₋₆₄. Alternatively, NPY whose amount is to be measured can be a degradation product of NPY₁₋₉₇, NPY₁₋₂₈, NPY₂₉₋₉₇, NPY₆₈₋₉₇, NPY₂₉₋₆₇, or NPY₂₉₋₆₄. Further alternatively, NPY whose amount is to be measured can be NPY₃₁₋₄₇, NPY₅₄₋₆₁, NPY₆₈₋₈₀, or NPY₈₁₋₈₈. Still further alternatively, NPY whose amount is to be measured can be a full-length NPY or a NPY fragment each including NPY₃₁₋₄₇, NPY₅₄₋₆₁, NPY₆₈₋₈₀, or NPY₈₁₋₈₈, or any part of NPY₃₁₋₄₇, NPY₅₄₋₆₁, NPY₆₈₋₈₀, or NPY₈₁₋₈₈.

The term "prostate cancer marker RNA" encompasses not only a full-length of the RNA described above, but also a fragment of the RNA and a precursor of the RNA. Note that TMPRSS2:ERG is fusion mRNA of TMPRSS2 gene and ERG gene. A manner how TMPRSS2 and ERG are fused to each other in TMPRSS2:ERG is not particularly limited. For example, TMPRSS2 and ERG can be fused to each other in a manner as described in the document "Jianghua Wang, Yi Cai, Chengxi Ren, and Michael Ittmann, Cancer Res 2006; 66: 8347-8351". PCA3 RNA is a transcription product of PCA3 gene, and is non-coding RNA.

The above-described prostate cancer marker peptide is detected in a greater amount particularly in sera derived from prostate cancer patients than in sera derived from healthy persons. Therefore, it is possible to detect prostate cancer by (i) measuring an existing amount of prostate cancer marker peptide particularly in a serum of a subject and (ii) comparing the existing amount of prostate cancer marker peptide with an existing amount of prostate cancer marker peptide measured in a serum of a healthy person. Further, the above-described prostate cancer marker RNA is detected in a greater amount particularly in urine derived from prostate cancer patients than in urine derived from healthy persons. Therefore, it is possible to detect prostate cancer by (i) measuring an existing amount of prostate cancer marker RNA particularly in urine of a subject and (ii) comparing the existing amount of prostate cancer marker RNA with an existing amount of prostate cancer marker RNA measured in urine of a healthy person.

Furthermore, NPY has an excellent specificity to prostate cancer, and an amount of NPY is highly relevant to a degree of progression of prostate cancer (see the later-described Example). Therefore, NPY is particularly suitable as a biomarker for assessing a degree of progression of prostate cancer.

Note here that the term "prostate cancer" refers to a malignant tumor that appears in a prostate gland and encompasses adenocarcinoma (prostatic adenocarcinoma).

The term "degree of progression" refers to a degree of progression or development determined based on criteria such as Gleason Score (GS), TNM classification, and/or a serum PSA level. Note that the "degree of progression" in the present invention can encompass not only (i) assessment in an extent of cancer progression or development in a subject having cancer but also (ii) assessment indicating that a subject does not have cancer. The expression "assessing a degree of progression of prostate cancer" can encompass not only (i) assessing, for a subject having prostate cancer, a degree of progression of the prostate cancer but also (ii) carrying out assessment to reach determination that a subject does not have prostate cancer. Namely, the expression "assessing a degree of progression of prostate cancer" can also encompass determining that "a subject does not have prostate cancer".

The term "sensitivity" refers to a percentage (true positive percentage) indicative of positive (abnormal subject value) observed in a case where an inspection is conducted on a group of subjects having a specific disease. The term "specificity" refers to a percentage (true negative percentage) indicative of negative (normal value) observed in a case where an inspection is conducted on a group of subjects not having a specific disease. The term "positive predictive value" refers to a percentage of individuals actually having a disease with respect to the subjects who have been determined to be positive in the inspection. The term "negative predictive value" refers to a percentage of individuals actually not having a disease with respect to the subjects who have been determined to be negative in the inspection.

Each of the analysis method and the assessment method according to the present invention only needs to include the step of measuring an amount of NPY in a sample derived from blood or urine obtained from a living organism, and is not particularly limited in terms of other specific steps, tools used therein, or devices used therein.

The expression "measuring an amount of prostate cancer marker" herein intends to measure an existing amount or a concentration of prostate cancer marker in a biological sample or in a sample obtained as a result of purification of the biological sample. The "amount of prostate cancer marker" can be an absolute amount (absolute mass or absolute concentration) or a relative amount (relative mass or relative concentration). For example, the "amount of prostate cancer marker" can be a peak area in mass spectrometry or a luminance intensity in luminance assay. Alternatively, the "amount of prostate cancer marker" may be a value indicative of how many times greater/smaller than a certain reference value.

The "sample" used in the present invention is a sample derived from blood or urine obtained from a living organism (subject). Examples of the sample derived from the blood include whole blood, a serum, and plasma. Among these, in order to measure an amount of NPY or an amount of PSA, the "sample" is preferably whole blood, a serum, or plasma, more preferably a serum or plasma. Among those listed above, in order to measure an amount of mRNA of TMPRSS2:ERG or an amount of PCA3 RNA, the "sample" is preferably urine.

The subject is, for example, a human, a mouse, a rat, a rabbit, or a monkey, and preferably a human. A site from which blood is collected is, for example, a median cubital vein, a cephalic vein, or a basilica vein. In order to measure amounts of various kinds of prostate cancer markers, either the same sample or different samples may be used.

Further, the subject can be the one who has been found to have prostate cancer or the one whose status as to whether he/she has prostate cancer is unknown. For the subject who has been found to have prostate cancer, it is possible to assess an extent of development of the prostate cancer. Meanwhile, for the subject whose status as to whether he/she has prostate cancer is unknown, it is possible to determine whether or not the subject has prostate cancer and to assess, if the subject has prostate cancer, an extent of development of the prostate cancer.

As an example of the assessment method according to the present invention, it is possible to assess a degree of progression of prostate cancer by (i) measuring an amount of NPY in a biological sample derived from a subject, (ii) measuring amounts of NPY in respective biological samples derived from control subjects (a healthy person and prostate cancer patients at various degrees of progression), and (iii) comparing the amount of NPY of in the sample derived from the subject with the amounts of NPY in the respective samples derived from the control subjects. Specifically, by comparing the amount of NPY in the sample derived from the subject with the amounts of NPY in the respective samples derived from the control subjects, it is possible to determine whether or not the subject has prostate cancer and to assess, if the subject has prostate cancer, a degree of progression of the prostate cancer in a statistic manner. The amounts of NPY in the respective samples derived from the control subjects can be the ones that have been measured in advance. Alternatively, the amounts of NPY in the respective samples derived from the control subjects can be measured in advance, and amount ranges for the respective degrees of progression can be predetermined based on the amounts thus measured. Further, if the amount of NPY in the biological sample derived from the subject is within one of the amount ranges, it is possible to determine that the degree of progression of the prostate cancer of the subject is at a degree of progression corresponding to the one of the amount ranges.

Further, by employing, as the control subject, a benign prostate hypertrophy (BPH) patient in addition to the healthy person and the prostate cancer patients at the various degrees of progression, it is possible to determine the degree of progression more accurately. This allows each of the analysis method and the assessment method according to the present invention to more accurately distinguish prostate cancer from BPH, which is particularly likely to be false-positive.

Note that the control subjects only need to include at least two prostate cancer patients of different degrees of progression, and may not include the healthy person or the BPH patient.

In comparing (i) the amount of NPY in the subject with (ii) the amounts of NPY in the control subjects, a significant difference therebetween can be determined by, for example, a statistical procedure such as t-test, F-test, chi-square test, or Mann-Whitney's U test.

Further, by carrying out the assessment based on NPY in combination of the other prostate cancer markers as those described above, it is possible to more accurately assess a degree of progression of prostate cancer.

In case of carrying out the assessment based on NPY and PSA in combination, for example, a logistic regression score is calculated based on amounts of NPY and PSA which has been measured. The calculation may be carried out according to, for example, the method described in the document "Pepe, M. S., Cai, T., and Longton, G. (2006), Biometrics 62, 221-229". Based on the logistic regression score, a degree of progression of prostate cancer is obtained. The combination of NPY and PSA is particularly favorable in terms of sensitivity and specificity. Thus, by the combination of NPY and PSA, it is possible to further more accurately assess a degree of progression of prostate cancer.

The logistic regression method can be applied not only to the combination of NPY and PSA but also to a combination of NPY and at least one of the other prostate cancer markers.

The method for measuring the amount of prostate cancer marker peptide is not particularly limited, as long as the method is able to determine the existing amount of prostate cancer marker peptide in a quantitative manner or a semiquantitative manner. For example, the method for measuring the amount of prostate cancer marker peptide can be (i) a method employing an immunological technique using an antibody specific to the prostate cancer marker peptide, (ii) a method using a peptide probe specific to the prostate cancer marker peptide, (iii) a liquid chromatography method, or (iv) mass spectrometry. Examples of the method using an antibody encompass ELISA, quantitative western blotting, radioimmunoassay, immunochromatography, and immunoprecipitation. For example, a type of ELISA can be, but is not particularly limited to, ELISA by means of direct absorption, competitive ELISA, sandwich ELISA, and ELISA for assaying a microvolume sample with use of a microfluidic system or microbeads, each of which is a so-called antigen assaying system (assaying an amount of antigen contained in a biological sample).

The antibody specific to the prostate cancer marker peptide can be a monoclonal antibody or a polyclonal antibody, and is preferably a monoclonal antibody. For example, amino acid sequences of human NPY and human PSA are available from public database such as UniProt. In the database UniProt, for example, human NPY is registered as accession number P01303, and human PSA is registered as accession number P07288. Based on this information, a person skilled in the art is able to easily determine an amino acid sequence suitable as an antigen for preparing an antibody specific to the prostate cancer marker peptide.

The term "antibody" in the present invention intends to include (i) immunoglobulin of all classes and all subclasses and (ii) a functional fragment of the antibody. The term "antibody" has a conception including each of a natural polyclonal antibody and a natural monoclonal antibody and also including an antibody produced by the genetic recombinant technology and a functional fragment of such an antibody. The term "functional fragment of the antibody" denotes a fragment including a partial region of any of the above-described antibodies and having an antigen binding capacity (synonymous with "antigen-binding fragment"). The natural antibody is not particularly limited to any type, and can be derived from any living things such as a human, a mouse, a rat, a monkey, a goat, a rabbit, a camel, a llama, a cow, and a chicken. The antibody produced by the genetic recombinant technology is not particularly limited to any type, and can be, for example, (i) a chimeric antibody such as a humanized or primatized antibody obtained as a result of genetic modification of a natural antibody, (ii) a synthesized antibody, (iii) a recombinant antibody, (iv) a mutated antibody, or (v) a graft-binding antibody (e.g., an antibody to which another protein, a radioisotopic label, and/or the like is/are conjugated or fused). In addition, the antibody produced by the genetic modification technology can also be an antibody obtained as a result of introducing, into an antibody produced by the genetic recombinant technology, the same modification as that introduced into the natural antibody as described above. Specific examples of the functional fragment of the antibody encompass F(ab')₂, Fab', Fab, Fv (variable fragment of antibody), sFv, dsFv (disulphide stabilized Fv), and dAb (single domain antibody).

Further, a concept of the term "antigen-binding fragment" in the present invention encompasses an antibody fragment which is mutated within a range allowing the fragment to maintain reactivity with a target polypeptide. The mutation is carried out by a known technique such as the genetic modification technique which can be appropriately selected by a person skilled in the art.

It is possible to detect the above-described antibody by causing the antibody to form a complex together with a secondary antibody labeled with a reporter molecule capable of attracting a detectable signal. Alternatively, it is possible to carry out the detection with use of the above-described antibody having been labeled with the reporter molecule in advance. The reporter molecule is, for example, an enzyme, a fluorophore-containing molecule, or a radionuclide-containing molecule. As such a reporter molecule, various kinds of molecules are known. A person skilled in the art can select any appropriate one among them.

In a case where the enzyme is used as the reporter molecule, the enzyme can be, for example, peroxidase, β-galactosidase, alkaline phosphatase, glucose oxidase, or acetylcholinesterase. It is possible to bind such an enzyme and the antibody to each other by a known method involving use of a crosslinker such as a maleimide compound. A substrate used therefor can be a known substance selected according to the type of the enzyme which is to be used. For example, in a case where peroxidase is used as the enzyme, it is possible to use 3,3',5,5'-tetramethylbenzidine, for example. Meanwhile, in a case where alkaline phosphatase is used as the enzyme, it is possible to use para-nitrophenol, for example. As the radionuclide, it is possible to use ¹²⁵I or ³H, for example. As a fluorescent dye, it is possible to use fluorescein isothiocyanate (FITC) or tetramethylrhodamine isothiocyanate (TRITC), for example.

For the detection, it is possible to employ a simple measurement system such as immunochromatography according to which the antibody is detected by agglutination of gold colloids coated with the secondary antibody.

Further, in the method involving use of the peptide probe, a peptide probe labeled with the above-described reporter molecule may be used, for example.

Thus, the amount of prostate cancer marker peptide can be measured by (i) luminance assay encompassing an assay for color or fluorescence or (ii) radioassay.

The mass spectrometry can be carried out with use of a known mass spectrometer. Since the mass spectrometry involving use of the mass spectrometer is excellent in sensitivity and accuracy, such mass spectrometry is able to carry out accurate determination. Further, with use of a mutlichannel mass spectrometer capable of simultaneous multicomponent analysis, it is possible to simultaneously measure amounts of two or more types of prostate cancer marker peptides. Furthermore, this makes it possible to simultaneously measure (i) the prostate cancer biomarker and (ii) a biomarker for another disease which is not the prostate cancer, thereby enabling to attempt to detect various diseases at once. In order to carry out the detection more accurately, it is preferable to use a tandem mass spectrometer (MS/MS). The mass spectrometer used in the detection method of the present invention is not particularly limited to any type, as long as the mass spectrometer is capable of determining a quantity. The mass spectrometer used in the detection method of the present invention can be a conventionally-known mass spectrometer such as a quadrupole mass spectrometer or a time-of-flight mass spectrometer.

Among those described above, the mass spectrometry or the luminance assay is preferable as the method for measuring the amount of prostate cancer marker peptide.

It is preferable that, prior to the measurement of the amount of prostate cancer marker peptide, (i) the sample used in the measurement is subjected to reversed phase extraction twice or more so that the sample is concentrated as a fraction containing NPY and then (ii) an amount of NPY is measured in the fraction. This allows to effectively remove a large protein (e.g., > 20 kDa) from the sample, thereby enhancing accuracy in the measurement of the amount of NPY. A fraction to be collected as the fraction containing NPY is not limited in terms of a range of a molecular weight, as long as the fraction contains NPY. For example, as the fraction to be collected, a fraction containing a peptide having a molecular weight of less than 20 kDa can be collected. Further, in order to measure an amount of another prostate cancer marker peptide which is not NPY in addition to the amount of NPY, the fraction to be collected can be selected according to the molecular weight of the another prostate cancer marker peptide. For the reversed phase extraction, it is preferable to use, for example, the QUEST-MS technology, which will be explained in the later-described Example.

In order to carry out the measurement by the mass spectrometry, the sample to be used in the measurement of the amount of prostate cancer marker peptide is preferably subjected to a denaturation treatment prior to the measurement of the amount of prostate cancer marker peptide after the sample is collected from a living organism. Examples of the denaturation treatment encompass addition of a chaotropic salt, acid denaturation, a heat treatment, and addition of a surfactant. Examples of a denaturation agent encompass urea and guanidine hydrochloride. Subsequently, the sample is preferably subjected to reduction and alkylation. The reduction and alkylation can be carried out by a known method. As a result of the denaturation, reduction, and alkylation, a disulfide bond between cysteines in the peptide is cut and blocked. This allows, in a digestive treatment carried out on the sample prior to the mass spectrometry, to carry out digestion sufficiently by a digestive enzyme such as trypsin.

Note that, in measurement of amounts of plural types of prostate cancer marker peptides, the prostate cancer marker peptides can be measured by respective different methods according to their types or by the same method.

The method for measuring the amount of prostate cancer marker RNA is not particularly limited to any kind, as long as the method is able to determine the existing amount of prostate cancer marker RNA in a quantitative manner or a semiquantitative manner. For example, the method for measuring the amount of prostate cancer marker RNA can employ a method of amplifying RNA by means of a nucleic-acid amplification technique such as PCR. Examples of the method involving use of the nucleic-acid amplification technique encompass reverse transcription PCR, real-time PCR, and quantitative RT-PCR. As a method for directly detecting RNA, northern blotting or the like can be employed. In the measurement of the amount of prostate cancer marker RNA, cDNA can be prepared by using, as a template, RNA contained in the biological sample.

Base sequences of human TMPRSS2:ERG and human PCA3 are available from a public database such as NCBI. For example, in the database NCBI, human TMPRSS2 is registered as accession number NM_005656, human ERG is registered as accession number NM_004449, and human PCA3 is registered as accession number NR_015342. An example of a manner how TMPRSS2 and ERG are fused to each other in TMPRSS2:ERG is described in, e.g., the document "Jianghua Wang, Yi Cai, Chengxi Ren, and Michael Ittmann, Cancer Res 2006; 66: 8347-8351". Based on this information, it is possible for a person skilled in the art to easily design a primer suitable to amplify mRNA of TMPRSS2:ERG or PCA3 RNA. Further, based on the above information, it is possible for a person skilled in the art to easily design a nucleic acid probe suitable to detect mRNA of TMPRSS2:ERG or PCA3 RNA by means of, e.g., northern blotting.

Note that, in measurement of amounts of plural types of prostate cancer marker RNAs, the prostate cancer marker RNAs can be measured by respective different methods according to their types or by the same method.

### [2. A method for detecting prostate cancer]

A method according to the present invention for detecting prostate cancer includes the step of measuring respective amounts of NPY and PSA in a sample derived from blood or urine obtained from a living organism.

One example of the detection method of the present invention is as follows. That is, by (i) measuring respective amounts of NPY and PSA in a biological sample derived from a subject, (ii) measuring respective amounts of NPY and PSA in a biological sample derived from a control subject (healthy person), and (iii) comparing the amounts measured in the subject with the amounts measured in the control subject, it is possible to detect prostate cancer. Specifically, for example, based on the amounts of NPY and PSA which have been measured, respective logistic regression scores of the subject and the control subject are calculated. The calculation may be carried out according to, for example, the method described in the document "Pepe, M. S., Cai, T., and Longton, G. (2006), Biometrics 62, 221-229". If a significant difference is found between the logistic regression scores thus calculated, it is possible to determine that the subject has prostate cancer or that the subject may have prostate cancer with a high possibility. The amount(s) of NPY and/or PSA in the sample derived from the control subject can be the one(s) that has/have been measured in advance. Alternatively, the amounts of NPY and PSA in the biological sample derived from the control subject can be measured in advance, a logistic regression score therefor can be calculated, and a normal value range of the logistic regression score can be determined based on the calculation result. Then, if the logistic regression score calculated for the biological sample derived from the subject is outside the normal value range, it is possible to determine that the subject has prostate cancer.

Further, by employing, as the control subject, a benign prostate hypertrophy (BPH) patient in addition to the healthy person, it is possible to more accurately determine whether or not the subject has prostate cancer. This allows the method according to the present invention for detecting prostate cancer to more accurately distinguish prostate cancer from BPH, which is particularly likely to be false-positive.

In comparing (i) the logistic regression score of the subject with (ii) the logistic regression score of the control subject, a significant difference therebetween can be determined by, for example, a statistical procedure such as t-test, F-test, chi-square test, or Mann-Whitney's U test.

The method for measuring the respective amounts of NPY and PSA is not limited to any kind, as long as the method is able to determine their existing amounts in a quantitative manner or a semiquantitative manner. For example, the measuring method can be any of the methods described in [1. An analysis method for assessing a degree of progression of prostate cancer and a method for assessing a degree of progression of prostate cancer] above.

In the method according to the present invention for detecting prostate cancer, a poor specificity of PSA is complemented by NPY effectively (see also the later-described Example). Further, in this method, a sensitivity of PSA is hardly decreased. Namely, this method provides good sensitivity and specificity, and therefore is able to detect prostate cancer more accurately. Particularly, the improvement in specificity enables to reduce overdiagnosis and unnecessary biopsies, thereby leading to alleviation of both physical and mental stress for men.

### [3. Test kit]

A test kit according to one aspect of the present invention is a test kit for assessing a degree of progression of prostate cancer, the test kit including a peptide probe for measuring an amount of NPY in a sample derived from blood or urine obtained from a living organism. The test kit according to the present invention preferably further includes at least one of (i) an antibody or a peptide probe each for measuring an amount of PSA in a sample derived from blood or urine obtained from the living organism, (ii) a nucleic-acid probe or a nucleic-acid primer each for measuring an amount of mRNA of TMPRSS2:ERG in a sample derived from blood or urine obtained from the living organism, and (iii) a nucleic-acid probe or a nucleic-acid primer each for measuring an amount of PCA3 RNA in a sample derived from blood or urine obtained from the living organism.

A test kit according to another aspect of the present invention is a test kit for assessing a degree of progression of prostate cancer, the test kit including an antibody for measuring an amount of neuropeptide Y in a sample derived from blood or urine obtained from a living organism, the test kit further including at least one of (i) an antibody or a peptide probe each for measuring an amount of prostate-specific antigen in a sample derived from blood or urine obtained from the living organism, (ii) a nucleic-acid probe or a nucleic-acid primer each for measuring an amount of mRNA of TMPRSS2:ERG in a sample derived from blood or urine obtained from the living organism, and (iii) a nucleic-acid probe or a nucleic-acid primer each for measuring an amount of PCA3 RNA in a sample derived from blood or urine obtained from the living organism.

The "peptide probe" denotes a peptidic probe capable of specifically binding to NPY or PSA. Specific examples of the peptide probe encompass a peptide sequence capable of specifically binding to NPY or PSA. The peptide probe included in the test kit may include a natural amino acid and/or an unnatural amino acid.

The "nucleic-acid probe" denotes a nucleic-acid probe capable of specifically binding to mRNA of TMPRSS2:ERG or PCA3 RNA. More specifically, the nucleic-acid probe may be TaqMan probe or Invader probe, for example. The "nucleic-acid primer" denotes a nucleic-acid primer capable of specifically amplifying (i) mRNA of TMPRSS2:ERG or PCA3 RNA or (ii) cDNA obtained as a result of reverse transcription of mRNA of TMPRSS2:ERG or PCA3 RNA. More specifically, the nucleic-acid primer may be a primer to be used in nucleic acid amplification, for example.

It is possible for a person skilled in the art to easily design each of the antibody, the peptide probe, the nucleic-acid probe, and the nucleic-acid primer based on information as to an amino acid sequence or a base sequence which information is obtained from the above-described public database.

As needed, the test kit for assessing a degree of progression of prostate cancer may further include at least one of, e.g., (i) any of various reagents (e.g., a secondary antibody, a reporter molecule, and a buffer) and various instruments (e.g., a plate and a pipet) each for immunologically detecting the antibody or the peptide probe, (ii) any of various reagents (e.g., a buffer) and various instruments (e.g., a plate and a pipet) each for detecting the nucleic-acid probe, (iii) any of various reagents (e.g., a polymerase, various dNTP, and a buffer) and various instruments (e.g., a tube and a pipet) each used in the nucleic acid amplification, (iv) an instruction manual of the test kit, (v) a sample serving as a control to be used in the measurement, and (vi) data serving as a control to be used in analysis of a result of the measurement. Note that the instruction manual of the test kit for assessing a degree of progression of prostate cancer includes the descriptions about the analysis method and/or the assessment method according to the present invention explained in [1. An analysis method for assessing a degree of progression of prostate cancer and a method for assessing a degree of progression of prostate cancer] above.

A test kit according to further another aspect of the present invention is a test kit for detecting prostate cancer, the test kind including: an antibody or a peptide probe each for measuring an amount of neuropeptide Y in a sample derived from blood or urine obtained from a living organism; and an antibody or a peptide probe each for measuring an amount of prostate-specific antigen in a sample derived from blood or urine obtained from the living organism.

As needed, the test kit for detecting prostate cancer may further include at least one of, e.g., (i) any of various reagents (e.g., a secondary antibody, a reporter molecule, and a buffer) and various instruments (e.g., a plate and a pipet) each for immunologically detecting the antibody or the peptide probe, (ii) an instruction manual of the test kit, (iii) a sample serving as a control to be used in measurement, and (iv) data serving as a control to be used in analysis of a result of the measurement. Note that the instruction manual of the test kit for detecting prostate cancer includes the description about the detection method according to the present invention explained in [2. A method for detecting prostate cancer] above.

Each of the test kit for assessing a degree of progression of prostate cancer and the test kit for detecting prostate cancer is preferably, but is not particularly limited to, a test kit based on ELISA or immunochromatography, for example.

### [4. Others]

A result obtained by carrying out (i) the analysis method or the assessment method explained in [1. An analysis method for assessing a degree of progression of prostate cancer and a method for assessing a degree of progression of prostate cancer] above or (ii) the detection method explained in [2. A method for detecting prostate cancer] above can be utilized as one of materials used by a doctor for diagnosis. Further, if it is determined that a subject has a possibility of prostate cancer as a result of carrying out (i) the analysis method or the assessment method explained in [1. An analysis method for assessing a degree of progression of prostate cancer and a method for assessing a degree of progression of prostate cancer] above or (ii) the detection method explained in [2. A method for detecting prostate cancer] above, the subject can receive a therapy also based on, as needed, diagnosis made by a doctor. Here, examples of the therapy encompass chemotherapy, radiotherapy, and a surgical operation carried out by a doctor or, depending on the situation, an expert who is not a doctor. Particularly, since the analysis method or the assessment method according to the present invention is able to assess a degree of progression of prostate cancer, it is possible to promptly decide a therapy suitable for the degree of progression.

More specifically, for example, in a case where a doctor diagnoses that a subject has "prostate cancer of a high degree of progression" based on a result obtained by carrying out the method according to the present invention, it is possible to carry out a prostate biopsy on the subject after consideration of (i) an observation by means of another inspection (e.g., a rectal examination, echography, and/or MRI scanning) and/or (ii) an age, a family history, and/or the like of the subject. Typically, a definitive diagnosis as to whether or not the subject has prostate cancer is made based on the result of the prostate biopsy, and thereafter a therapeutic strategy for the subject is decided. A doctor can diagnose that a subject has "prostate cancer of a high degree of progression" in the following manner. First, a certain reference value is predetermined. Then, if a value measured in the subject is equal to or higher than the reference value, the doctor can diagnose that the subject has "prostate cancer of a high degree of progression".

Meanwhile, if a doctor diagnoses that a subject "does not have prostate cancer" or has "prostate cancer of a low degree of progression" based on a result obtained by carrying out the method of the present invention, the doctor can carry out, after the diagnosis, follow-up observations by measuring the prostate cancer marker such as NPY and/or PSA periodically. Note that the number of degrees of progression of prostate cancer in the assessment is not limited to two, "high" and low". Alternatively, the assessment can be made in a larger number of degrees of progression.

As indicated in the later-described Example, each of fibronectin and electron transfer flavoprotein-ubiquinone dehydrogenase is also identified as a candidate prostate cancer biomarker. Therefore, it is possible to assess a degree of progression of prostate cancer and to detect prostate cancer by using (i) fibronectin and/or electron transfer flavoprotein-ubiquinone dehydrogenase and (ii) NPY and/or PSA in combination. In this case, each of the assessment method and the detection method can be carried out in the same manner as that described above.

### [5. Summary]

As described above, an analysis method according to the present invention for assessing a degree of progression of prostate cancer includes the step of measuring an amount of neuropeptide Y in a sample derived from blood or urine obtained from a living organism.

The analysis method according to the present invention for assessing a degree of progression of prostate cancer preferably further includes the step of measuring an amount of at least one of prostate-specific antigen, mRNA of TMPRSS2:ERG, and PCA3 RNA in a sample derived from blood or urine obtained from the living organism.

The analysis method according to the present invention for assessing a degree of progression of prostate cancer preferably further includes the step of measuring an amount of prostate-specific antigen in a sample derived from blood or urine obtained from the living organism.

The analysis method according to the present invention for assessing a degree of progression of prostate cancer can be arranged such that the amount of neuropeptide Y is measured by mass spectrometry or luminance assay.

The analysis method according to the present invention for assessing a degree of progression of prostate cancer is preferably arranged such that the sample is whole blood, a serum, or plasma.

The analysis method according to the present invention for assessing a degree of progression of prostate cancer can be arranged such that, in the step of measuring the amount of neuropeptide Y, the sample is subjected to reversed phase extraction twice or more so that a fraction containing neuropeptide Y is obtained, and the amount of neuropeptide Y is measured in the fraction thus obtained.

A method according to the present invention for detecting prostate cancer includes the step of measuring respective amounts of neuropeptide Y and prostate-specific antigen in a sample derived from blood or urine obtained from a living organism.

A method according to the present invention for assessing a degree of progression of prostate cancer includes the step of measuring an amount of neuropeptide Y in a sample derived from blood or urine obtained from a living organism.

A test kit according to one aspect of the present invention is a test kit for assessing a degree of progression of prostate cancer, the test kit including a peptide probe for measuring an amount of neuropeptide Y in a sample derived from blood or urine obtained from a living organism.

The test kit according to the one aspect of the present invention preferably further includes at least one of (i) an antibody or a peptide probe each for measuring an amount of prostate-specific antigen in a sample derived from blood or urine obtained from the living organism, (ii) a nucleic-acid probe or a nucleic-acid primer each for measuring an amount of mRNA of TMPRSS2:ERG in a sample derived from blood or urine obtained from the living organism, and (iii) a nucleic-acid probe or a nucleic-acid primer each for measuring an amount of PCA3 RNA in a sample derived from blood or urine obtained from the living organism.

A test kit according to another aspect of the present invention is a test kit for assessing a degree of progression of prostate cancer, the test kit including: an antibody for measuring an amount of neuropeptide Y in a sample derived from blood or urine obtained from a living organism, the test kit further including at least one of (i) an antibody or a peptide probe each for measuring an amount of prostate-specific antigen in a sample derived from blood or urine obtained from the living organism, (ii) a nucleic-acid probe or a nucleic-acid primer each for measuring an amount of mRNA of TMPRSS2:ERG in a sample derived from blood or urine obtained from the living organism, and (iii) a nucleic-acid probe or a nucleic-acid primer each for measuring an amount of PCA3 RNA in a sample derived from blood or urine obtained from the living organism.

A test kit according to further another aspect of the present invention is a test kit for detecting prostate cancer, the test kit including: an antibody or a peptide probe each for measuring an amount of neuropeptide Y in a sample derived from blood or urine obtained from a living organism; and an antibody or a peptide probe each for measuring an amount of prostate-specific antigen in a sample derived from blood or urine obtained from the living organism.

The embodiments of the present invention will be described in further detail via the following Example. Needless to say, the present invention is not limited to the Example. It will be clear that the invention being thus described may be varied in many ways. Further, the present invention is not limited to the description of the embodiments above, but may be altered by a skilled person within the scope of the claims. An embodiment based on a proper combination of technical means disclosed in different embodiments is encompassed in the technical scope of the present invention. Furthermore, all the documents described herein are incorporated therein by reference.

### [Example]

### [Experiment procedure]

### (Reagents)

Tris(2-carboxyethyl)phosphine (TCEP), iodoacetamide, and ammonium bicarbonate were purchased from Sigma-Aldrich. Urea was purchased from GE Healthcare. Trypsin Gold was supplied by Promega. Trifluoroacetic acid (TFA) was purchased from Shimadzu Corporation.

### (Plasma samples)

EDTA-plasma samples (n = 116, Table 1) for biomarker screening were collected in Kochi Medical School Hospital. These samples were collected via blood collection from arm veins.

**[Table 1]**

| Group | n | Average Age | Sex |
|---|---|---|---|
| Healthy control | 24 | 69.1 | Male |
| BPH* | 19 | 68.9 | Male |
| PCa** (GS*** 5 to 6) | 20 | 65.3 | Male |
| PCa** (GS*** 7) | 28 | 69.7 | Male |
| PCa** (GS*** 8 to 10) | 25 | 68.1 | Male |
| Total | 116 | 68.2 | Male |

| | | | |
|---|---|---|---|
| *: "BPH" stands for "benign prostate hypertrophy". **: "PCa" stands for "prostate cancer". ***: "GS" stands for "Gleason score". | | | |

For a validation experiment for a biomarker, plasma samples from 26 healthy persons were collected as controls in Cancer Screening Center in the Cancer Institute Hospital of Japanese Foundation for Cancer Research (JFCR). Further, for the validation experiment, plasma samples from 19 benign prostate hyperplasia (BPH) patients and 65 prostate cancer patients were collected in Kochi Medical School Hospital, Kyoto University Hospital, and Iwate Medical University Hospital (Table 2). These samples were collected via blood collection from arm veins.

**[Table 2]**

| Group | n | Average Age | Sex |
|---|---|---|---|
| Healthy control | 26 | 69.1 | Male |
| BPH* | 19 | 68.9 | Male |
| PCa** (GS*** 5 to 6) | 17 | 65.9 | Male |
| PCa** (GS*** 7) | 30 | 64.2 | Male |
| PCa** (GS*** 8 to 10) | 18 | 66.1 | Male |
| Total | 110 | 64.0 | Male |

| | | | |
|---|---|---|---|
| *: "BPH" stands for "benign prostate hypertrophy". **: "PCa" stands for "prostate cancer". ***: "GS" stands for "Gleason score". | | | |

The experimental procedure was fully explained to all the patients above, and written informed consent was obtained from all the patients above. The experiment described below was approved by the following individual institutional ethical committees: the Ethical Committee of RIKEN; and the Institutional Review Boards of Kochi Medical School, JFCR, Kyoto University, and Iwate Medical University.

### (QUEST-MS purification)

On a 96-well polypropylene plate, 20 µl of each plasma sample was mixed with 80 µl of 10M urea in 50 mM ammonium bicarbonate. Subsequently, the sample was subjected to reduction with 5 mM TCEP at 37°C for 15 minutes, and then to alkylation with 25 mM iodoacetamide at room temperature for 15 minutes. Thereafter, the sample was diluted 4 times with 50 mM ammonium bicarbonate, and was loaded onto an equilibrated Oasis HLB 96-well µElution plate (2-mg sorbent per well, 30 µm particle size, available from Waters Corporation). Here, all procedures on the solid phase extraction plate were carried out by a custom-made 96-well syringe robot.

The Oasis plate was pre-wetted with 500 µl of 70% acetonitrile, and then equilibrated with 500 µl of 0.1% TFA in 2% acetonitrile at a rate of 250 µl/min. The sample was loaded at a rate of 100 µl/min, and thereafter the plate was washed twice with 500 µl of 0.1% TFA in 2% acetonitrile at a rate of 250 µl/min. Proteins were eluted therefrom with 100 µl of 40% acetonitrile at a rate of 100 µl/min, and the sample was subsequently diluted 5 times with 0.1% TFA prior to second purification with the Oasis plate. The sample thus diluted was processed under the same conditions as those for the first purification.

The eluate was lyophilized by a vacuum spin dryer, followed by digestion with 8 ng/µl Trypsin Gold (available from Promega) in 50 mM ammonium bicarbonate at 37°C for 6 hours. The digestive reaction was quenched by addition of 50 µl of 1.2% TFA in 4% acetonitrile.

### (LC/MS/MS analysis)

After the above-described QUEST-MS purification, 1 µl of sample was analyzed by a mass spectrometer (LTQ-Orbitrap-Velos mass spectrometer, available from Thermo Fisher Scientific Inc.) equipped to Ultimate 3000 nano-flow HPLC (available from Thermo Fisher Scientific Inc.). Using 0.1% formic acid as solvent-A and 0.1% formic acid in acetonitrile as solvent-B, peptides were separated from the sample on C18 Chip-column (75 µm × 200 mm, available from Nikkyo Technos), where (i) a flow rate was 250 nL/min and (ii) a gradient of solvent-B was 2% to 30% for 95 minutes and 30% to 95% for 15 minutes. The peptides thus eluted were ionized with a spray voltage of 2000V, and MS data was obtained by a data-dependent fragmentation method in which the survey scan was carried out between m/z 400 and m/z 1600 at a resolution of 60,000 with automatic gain control (AGC) target value of 1.0 × 10⁶ ion counts. The top-20 intense precursor ions in each survey scan were subjected to low-resolution MS/MS obtainment in a normal CID scan mode with AGC target value of 5,000 ion counts in the linear ion trap.

### (Label-free quantification by Expressionist RefinerMS)

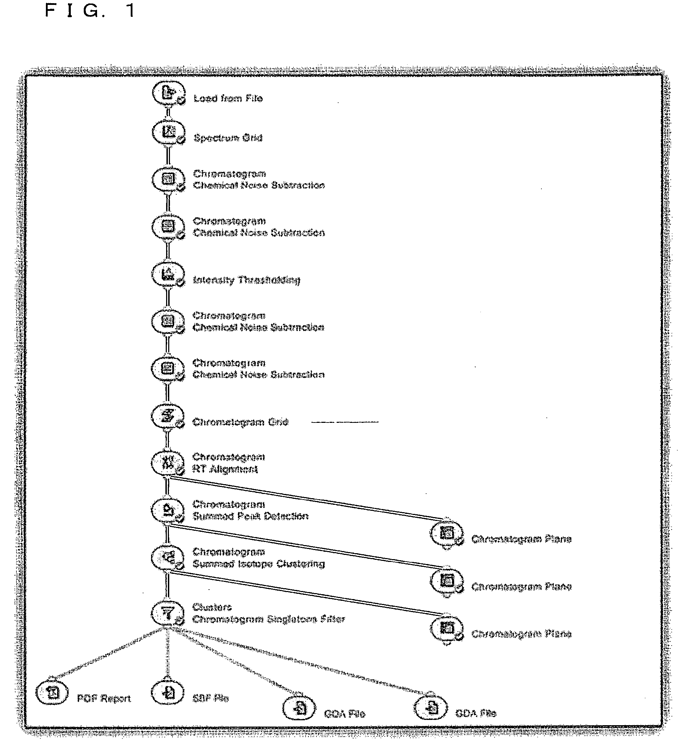

The raw data from LTQ-Orbitrap-Velos mass spectrometer was loaded onto Expressionist RefinerMS module (available from Genedata AG). The Expressionist RefinerMS module works, on an in-house server system, for data processing and label-free quantification analysis subsequently carried out. Fig. 1 shows an overall workflow of RefinerMS software. The Spectrum Grid was set at every 10 data points on 2D MS chromatogram planes (x = m/z, y = RT), and subsequently the first chemical noise subtraction was carried out with use of the following parameters: RT Window = 500 scans and Quantile = 80. Chromatogram smoothing was carried out by a moving average estimator for every 3 RT scans in the second chemical noise subtraction, and then signals having an intensity of less than 1000 were eliminated. The third and fourth chemical noise subtractions were applied to data using RT structure removal at a minimum RT length = 8 scans and m/z structure removal at a minimum m/z length = 4 points, respectively. The Chromatogram Grid was set at every 10 scans on the noise-subtracted data, followed by Chromatogram RT Alignment with use of the following parameters: m/z Window = 10 points, RT Window = 10 scans, Gap Penalty = 1, RT Search Interval - 2 minutes, and Alignment Scheme = pairwise alignment based tree. Next, the peaks on a temporarily-averaged chromatogram were detected by Summed Peak Detection Activity with use of the following parameters: Summation Window = 2 minutes, Overlap = 50, Minimum Peak Size = 10 scans, Maximum Merge Distance = 4 data points, Gap/Peak Ratio = 10, Method = curvature-based peak detection, Peak Refinement Threshold = 5, and Consistency Filter Threshold = 0.6. Finally, isotopic peaks derived from a single molecule were grouped into an isotope cluster by Summed Isotope Clustering Activity with use of the following parameters: Minimum Charge = 1, Maximum Charge = 8, Maximum Missing Peaks = 0, First Allowed Gap Position = 3, Ionization = protonation, RT Tolerance = 0.1 minute, m/z Tolerance = 0.05 Da, and Minimum Cluster Size Ratio = 0.5.

### (Extraction of biomarkers by Expressionist Analyst)

Since specificity of a new biomarker set should be maximized, 153,057 non-redundant detected peptides were filtered by an absent-present search algorithm. The absent-present search algorithm extracts peptides exhibiting all-or-nothing feature between two groups (healthy control + BPH vs. prostate cancer). Peptides detected in at most 1 case among 43 controls and at least 11 cases among 73 prostate cancer patients were selected, and were subjected to further validation experiments.

### (Protein identification)

The SEQUEST database search was performed by Proteome Discoverer 1.3 software (available from Thermo Fischer Scientific). The MS/MS data was searched in the human protein database SwissProt 2012_09 (20,235 sequences) with use of the following search parameters: Enzyme Name = Trypsin, Precursor Mass Tolerance = 3 ppm, Fragment Mass Tolerance = 0.8 Da, Dynamic Modification = Oxidation (M), and Static Modification = Carbamidomethyl (C). Peptide identifications that satisfied a false discovery rate (FDR) of less than 1% in SEQUEST Decoy Database Search were accepted. The raw data is published on the PRIDE database (http://www.ebi.ac.uk/pride/).

### (Multiple reaction monitoring (MRM) for NPY)

The plasma samples were processed with QUEST-MS procedure in the same manner as above, excepting that BSA tryptic digest of a final concentration of 1 fmol/µl was added to the samples as an internal control. Subsequently, the samples were analyzed by 4000 QTRAP mass spectrometer (available from AB Sciex) of Agilent 1200 nano-flow HPLC system (available from Agilent Technologies). With use of solvent-A (0.1% formic acid) and solvent-B (0.1% formic acid in acetonitrile), peptides were separated from the samples on C18 Chip-column (75 µm × 200 mm, available from Nikkyo Technos), where (i) a flow rate was 250 nL/min and (ii) a gradient of solvent B was a two-step linear gradient of 2% to 30% for 10 minutes and 30% to 95% for 5 minutes. MRM transitions specific to NPY and BSA (available from KYA technologies) were simultaneously monitored in the MRM mode of Analyst 1.5 software (available from AB Sciex). The instrumental settings were as follows: Ionization spray voltage = 2200V, Curtain gas (N₂) = 12 psi, CAD = 4, Declustering potential = 70V, Entrance potential = 10V, Q1 resolution = HIGH, Q3 resolution = LOW, and Pause in between = 2 msec. The MRM chromatograms thus obtained were analyzed by MultiQuant 2.02 software (available from AB Sciex). The mass chromatogram of each transition was smoothed by 1 pt window, and quantified for a peak area. After collision energy was optimized for 12 NPY transitions, quantification was substantially carried out only with Q1/Q3 = 466.23/272.20, which corresponds to NPY₈₁₋₈₈ peptide, because this transition showed the highest sensitivity. Then, with use of a BSA peptide which was measured in the same manner, an area of NPY₈₁₋₈₈ peak was normalized as follows: Normalized NPY = Area (NPY₈₁₋₈₈, Q1/Q3 = 466.2/272.2) / Area (BSA₆₆₋₇₅, Q1/Q3 = 582.3/951.5) × 10000.

### (Silver staining and image analysis)

In order to evaluate the efficacy of QUEST-MS purification, one-fifth of the purified sample was separated on 16% Tris-tricine SDS-PAGE gel (available from Life Technologies). The gel was stained with SilverQuest Silver Staining kit (available from Life Technologies) according to the manufacturer's instructions. The gel thus stained was scanned by GS-800 Calibrated Densitometer (available from Bio-Rad Laboratories), and analyzed by Image J software.

### (Immunohistochemical staining)

Immunohistochemical study was carried out with use of Ventana automated immunohistochemical systems (available from Ventana Medical Systems). For the study, slice sections (fixed by formalin and embedded into paraffin) of surgical or biopsy specimens collected from eight prostate cancer patients were used. The eight prostate cancer patients included three patients having prostatic intraepithelial neoplasia (PIN) lesions. The slice sections were incubated with a 1:400 diluted solution of polyclonal anti-NPY antibody (available from Abcam) for 16 minutes. The automated protocol was based on an indirect biotin-avidin system involving use of (i) a biotin-labeled secondary antibody and (ii) a diaminobenzidine substrate with hematoxylin counterstaining. The specificity of binding was confirmed by negative staining involving use of a rabbit nonimmune serum serving as a primary antibody.

### [Results]

### (Enrichment of low-molecular-weight polypeptides by QUEST-MS technology)

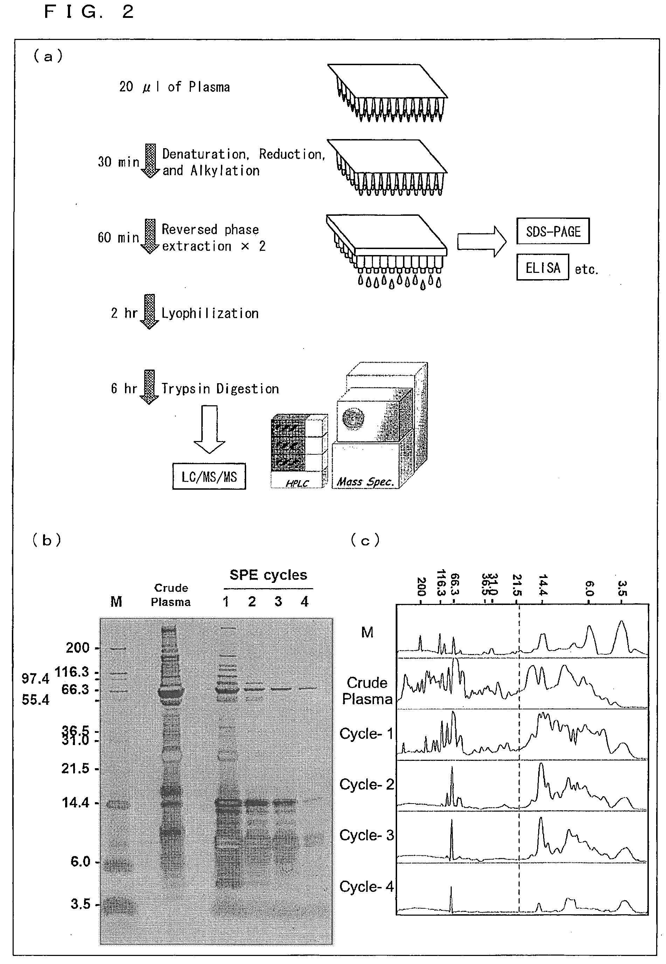

In order for accurate biomarker screening and seamless clinical application by targeting small polypeptides in blood, a pre-analytical purification method exhibiting high sensitivity, high reproducibility, high throughput, low cost, and ease of operation is necessary. In order to attain a method satisfying these criteria, the inventors of the present invention established a simple and effective enrichment method for proteins of less than 20 kDa in plasma ((a) of Fig. 2), and named the method "quick enrichment of small targets for mass spectrometry (QUEST-MS)". The QUEST-MS technology includes denaturation, reduction, alkylation, and subsequent tandem reversed phase extraction of crude plasma samples, for which it took only 1.5 hours per 96 samples.

The dried QUEST-MS-purified samples can be directly used in a protein assay such as SDS-PAGE or ELISA. Further, the QUEST-MS-purified samples can be subjected also to mass spectrometry after trypsinization.

In order to visualize the efficiency of enrichment of low-molecular-weight proteins by first to fourth reversed phase extractions with the Oasis HLB plate, crude plasma and purified proteins at each reversed phase extraction stage were separated on 16% Tris-tricine gel and stained with silver. The results thereof are shown in (b) of Fig. 2. The gel was subjected to quantitative desintometry analysis, and the results thereof are shown in (c) of Fig. 2. The quantitative density chromatograms of five lanes on the stained gel showed effective exclusion of > 20 kDa proteins at every solid phase extraction step. In particular, as a result of the second reversed phase extraction with the Oasis HLB plate, a percentage of low-molecular-weight proteins of less than 20 kDa exceeded 90%. The principle of such easy and clear-cut separation of small proteins is based on (i) pouring away huge proteins larger than a pore diameter (8 nm) and (ii) preventing middle-range proteins (20 kDa to 100 kDa) from being eluted by an excess absorption strength with respect to hydrophobic residues on the Oasis polymer beads. By repeatedly carrying out the reversed phase extraction three or more times, it is possible to purify the low-molecular-weight proteins more highly. However, for the purpose of retaining advantages in throughput and cost, the inventors of the present invention used the samples which had been purified twice for a further search and a further validation experiment for a biomarker.

### (Screening for low-molecular-weight biomarker for prostate cancer)

In order to avoid any unevenness among sampling by the institutes, the 116 plasma samples analyzed in the biomarker screening (Table 1) were collected in a single hospital, and strictly-controlled standard operation procedures were employed. Prior to QUEST-MS purification, 20 µl of each plasma sample underwent a single freeze-thaw step. LC/MS/MS analysis was conducted on the 116 purified samples, so that 153,057 non-redundant peptides were detected. The peptides thus detected were quantified by the Expressionist RefinerMS module in the above-described manner. In particular, this low-molecular-weight proteome catalog included four PSA-derived peptides of I₂₅VGGWECEK₃₃ (m/z = 539.25, z = +2), H₃₄SQPWQVLVASR₄₅ (m/z = 469.92, z = +3), A₄₈VCGGVLVHPQWVLTAAHCIR₆₈ (m/z = 782.08, z = +3), and L₁₂₆SEPAELTDAVK₁₃₇ (m/z = 636.84, z = +2). These four peptides were detected in 4 cases, 3 cases, 4 cases, and 5 cases from the control group (healthy controls + BPH patients), respectively. Meanwhile, these four peptides were detected in 15 cases, 13 cases, 10 cases, and 11 cases from the prostate cancer group, respectively. Based on values of the PSA test provided by the hospital, a detection limit of PSA according to the method of the inventors of the present invention could be estimated at approximately 10 ng/ml. This result suggests that general comparative tests (e.g., t-test or ANOVA) based on an average of samples are not appropriate for the extraction of specific biomarkers, because valid values of low-concentration tumor markers in the control group should be quite few. In general, a detection frequency of ideal tumor markers would be near 0 in the control samples, which will be exempted from application of t-test or ANOVA.

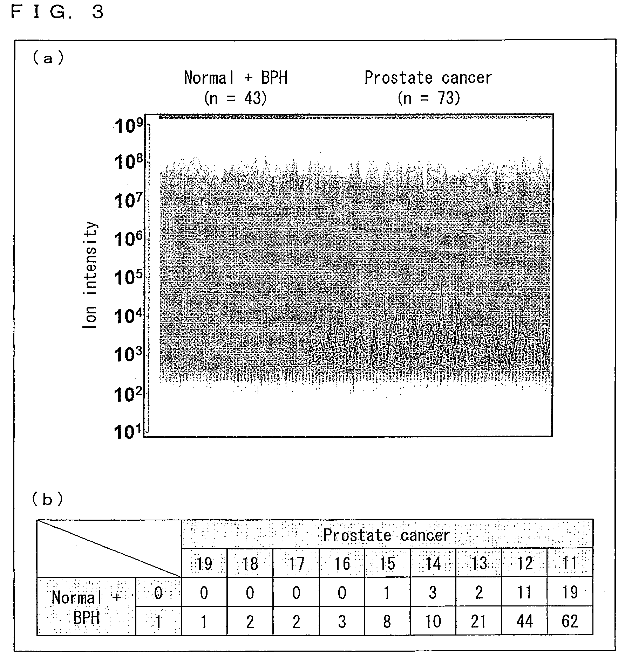

Subsequently, in order to maximize the specificity of the new biomarker set, the absent-present search algorithm was employed. This search resulted in identification of 189 peptides, which were detected in 0 or 1 case among 43 controls (24 healthy controls + 19 BPH patients) and were detected in at least 11 cases among 73 prostate cancer patients. In (a) of Fig. 3, 189 peptides among all of the 153,057 peptides exhibits a detection pattern specific to prostate cancer, and these 189 peptides were detected only in 0 or 1 case among the control group. Here, quantitative information of the 189 peptides is shown by black dots or lines on a two-dimensional plane. In (a) of Fig. 3, the x-axis indicates 110 individuals, whereas the y-axis indicates a peak strength in LC/MS/MS. (b) of Fig. 3 shows classifications of the 189 candidate biomarker peptides according to their detection frequencies. For example, 62 peptides were detected in 11 prostate cancer samples, and were detected in 1 control sample (healthy control or BPH).

The peptides identified as described above could be considered as candidate biomarkers which provide a lower false positive rate in addition to a similar or higher sensitivity in the diagnosis of prostate cancer patients as compared to those of PSA. Meanwhile, as a result of SEQUEST database search analysis (FDR < 0.01), 1,126 non-redundant plasma proteins were identified from 116 screening samples. The 189 candidate biomarker peptides were matched against the protein identification data set, so that three peptides listed in Table 3 were successfully identified. Considering the fact that expression of mRNA is specific to cerebral nervous system and prostate gland, neuropeptide Y (NPY) was used to next validation experiments.

**[Table 3]**

| UniProt accession number | UniProt ID | Protein name | Sequence* | Detection frequency in absent-present search | |
|---|---|---|---|---|---|
| | | | | Control* * | PCa*** |
| P01303 | NPY_HUMAN | Pro-neuropeptide Y | S68SPETLISDLLMR⁸⁰ | 0 | 11 |
| P02751 | FINC_HUMAN | Fibronectin | Q¹⁰⁴¹YNVGPSVSK¹⁰⁵⁰ | 1 | 11 |
| Q16134 | ETFD_HUMAN | Electron transfer flavoprotein ubiquinone oxidoreductase | G²¹³IATNDVGIQK²²³ | 1 | 11 |

| | | | | | |
|---|---|---|---|---|---|
| *: Numbers in the sequences indicate the amino acid numbers. **: The number of cases detected among the healthy controls or the BPH patients. ***: The number of cases detected among the prostate cancer patients. | | | | | |

### (NPY expression in prostate cancer tissues)

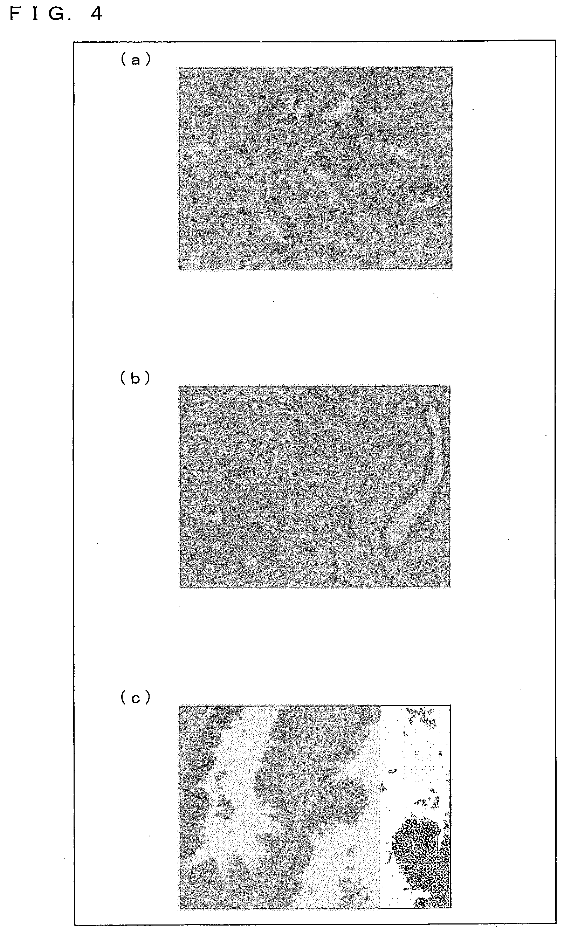

Fig. 4 shows the results of immunohistochemical staining of NPY. (a) and (b) of Fig. 4 show representative NPY immunostaining patterns in prostate cancer. The Gleason Score in (a) of Fig. 4 was 3 + 4, whereas that in (b) of Fig. 4 was 4 + 5. Immunoreactivity with anti-NPY antibody was observed in prostate cancer tissues. As a result, a strong immunoreactivity was observed in the cytoplasm of prostate cancer cells, whereas a weak immunoreactivity was observed in noncancerous prostate epithelium (the right side in (b) of Fig. 4). (c) of Fig. 4 indicates that the cytoplasmic immunoreactivity with anti-NPY was observed also in precursor cells of high-grade prostatic intraepithelial neoplasia (PIN). These results suggest that neoplastic lesions in prostate exhibit a high NPY expression.

### (MRM-based replication analysis for NPY)

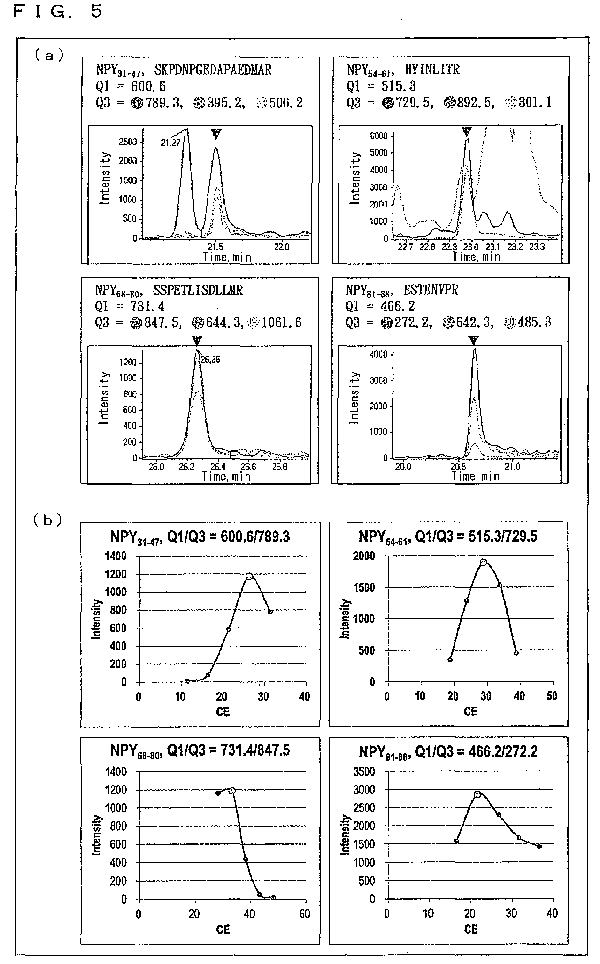

In order to evaluate a variation between different hospitals or different analytical methods, a further validation experiment for NPY was conducted with use of (i) an independent sample set and (ii) another mass spectrometric quantification MRM. In this experiment, healthy controls from JFCR were used instead of the ones from Kochi University hospital, and prostate disease samples from Kyoto University and Iwate Medical University were used (Table 2). The 110 samples were purified by QUEST-MS, which were then analyzed by 4000 QTRAP mass spectrometer by targeting four different peptides derived from NPY. Prior to MRM measurement, an optimum transition and collision energy (CE) capable of providing a highest peak intensity in MRM chromatogram were first searched among three transitions for each of the four NPY peptides. The results of the search are shown in (a) and (b) of Fig. 5. The results show that a transition Q1//Q3 = 466.2/272.2 for NPY⁸¹⁻⁸⁸ at CE = 17 eV was a reporter ion pair which was most sensitive to NPY. In (a) of Fig. 5, the figures shown on the right sides of Q1 and Q3 indicate the m/z settings in quadrupoles 1 and 3, respectively.

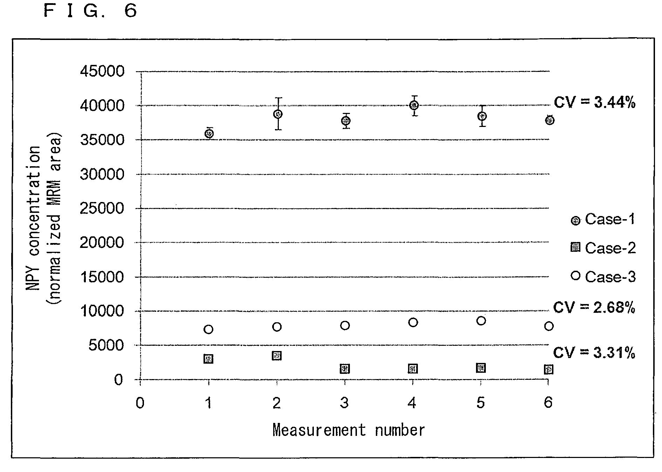

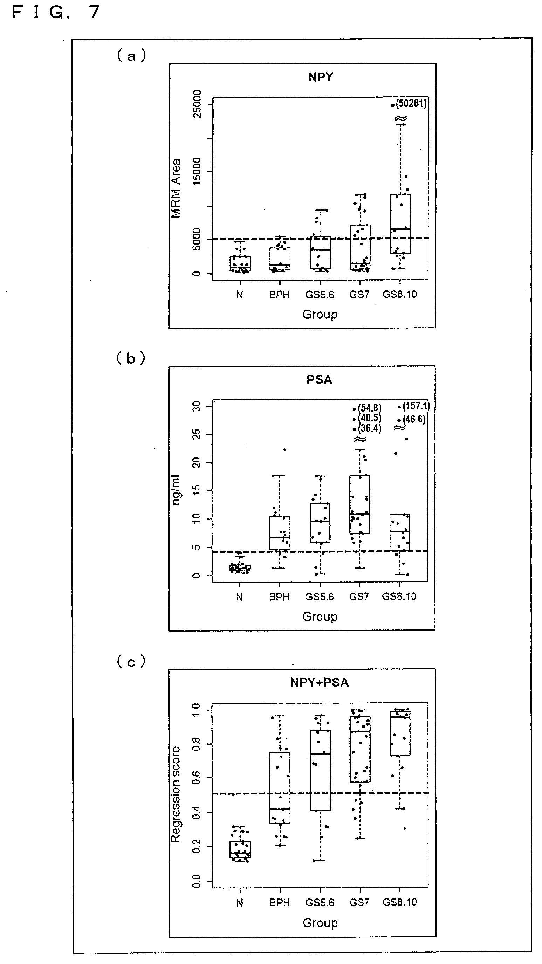

Further, 10 fmol of digested BSA was added to each sample so as to be used as an internal control for normalizing an inter-analytical variation. Subsequently, three plasma samples were analyzed at six different time points so as to evaluate quantitative reproducibility of MRM-based NPY measurement. The results of the analysis are shown in Fig. 6. In Fig. 6, each error bar indicates a standard deviation of three independent measurements. As shown in Fig. 6, a coefficient of variation (CV) was consistently smaller than 3.5%. Under the optimized MRM conditions described above, NPY in plasma was quantified in 110 cases. The results of the quantification are shown by the box plots in (a) of Fig. 7. Further, a concentration of PSA was quantified by ELISA, the results of which are shown in (b) of Fig. 7. These results show that NPY exhibits a higher diagnostic specificity particularly between BPH patients and prostate cancer patients of a Gleason score (GS) of 5 to 6 (p = 0.0396, t-test), as compared with PSA test (p = 0.2746, t-test). This observation confirmed the prostate-cancer-specific detection of NPY in the absent-present search analysis at the biomarker screening study.

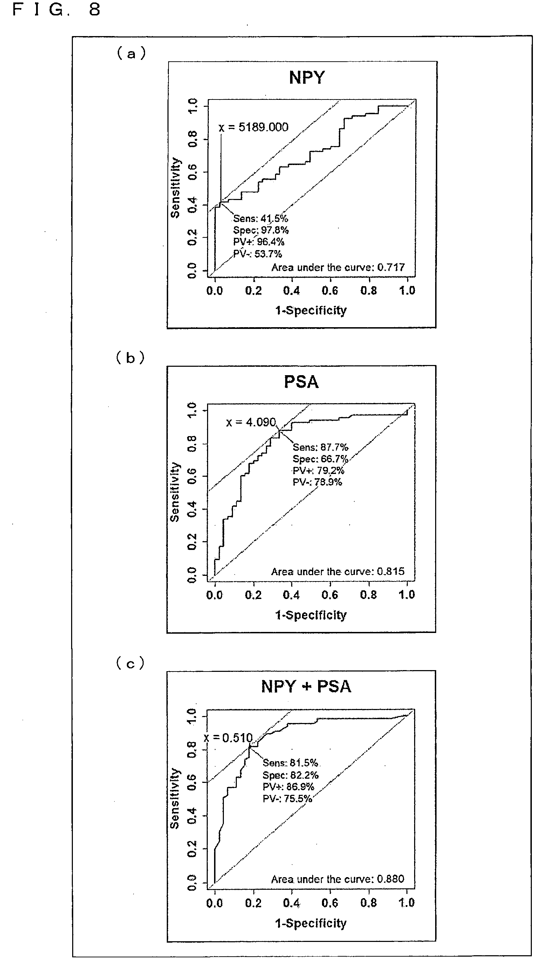

In Fig. 7, each broken line indicates a threshold between the control group (N + BPH) and the prostate cancer group. This threshold was calculated by an ROC curve analysis, which is illustrated in Fig. 8 (described later). For a largely deviated value, a specific figure indicative of a concentration corresponding to the value is described in a region above its respective box plot.

Thus, NPY involves a high specificity. However, in order to further enhance the sensitivity, the inventors of the present invention aimed to establish a diagnostic system involving use of a combination of NPY and PSA by a logistic regression method ((c) of Fig. 7). The logistic regression scores were calculated so that an area (AUC) under an ROC curve was maximized (according to the document "Pepe, M. S., Cai, T., and Longton, G. (2006), Biometrics 62, 221-229."). (a) though (c) of Fig. 8 show comparison between three ROC curves generated for NPY (an area of MRM chromatogram), PSA (values measured by ELISA in the hospitals), and NPY + PSA (logistic regression scores) in 110 plasma samples, respectively. In Fig. 8, a point of contact indicated by "x =" indicates an optimum threshold given by each marker. Further, in Fig. 8, "Sens" denotes a sensitivity, "Spec" denotes a specificity, "PV+" denotes a positive predictive value, and "PV-" denotes a negative predictive value.

The sensitivity and specificity of PSA by which 65 prostate cancer patients were distinguished from 45 controls (25 healthy controls and 20 BPH patients) were 87.7% and 66.7%, respectively. Meanwhile, such sensitivity and specificity of the combination of NPY and PSA were 81.5% and 82.2%, respectively. This result clearly shows that NPY is able to complement the poor specificity of PSA test without causing significant impairment of the high sensitivity of PSA test. Thus, the combination of NPY and PSA makes it possible to reduce overdiagnosis and unnecessary biopsies, thereby leading to alleviation of both physical and mental stress for men.

Furthermore, the above result shows that NPY is highly relevant to a degree of progression of prostate cancer and thus NPY is solely or in combination with PSA capable of assessing the degree of progression more accurately.

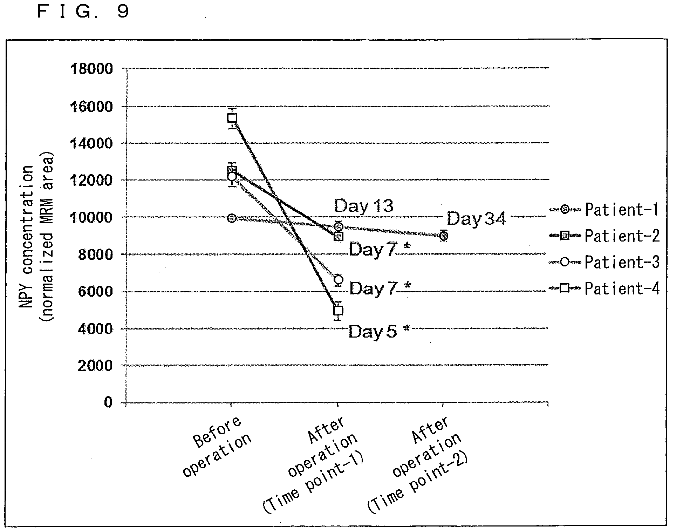

Moreover, in order to verify effectiveness of NPY as to evaluation of a treatment response, samples were collected from four prostate cancer patients before and after a surgical operation, and the samples thus collected were analyzed. The samples were subjected to MRM analysis three times, the results of which are shown in Fig. 9. In Fig. 9, the day counts indicate a period that had passed since transurethral resection of prostate (TURP). In Fig. 9, each error bar indicates a standard deviation of three independent measurements. As shown in Fig. 9, three (patients-2 through -4) of the four patients exhibited a reduction in plasma NPY level (*p < 0.05, Student's t-test) 5 to 7 days after the operation. Particularly, the NPY level in patient-4 was reduced to a value lower than a cut-off value (5189.0) 5 days after the operation. On the other hand, patient-1 exhibited only a slight change in NPY level even one month after the operation. The sustained high NPY concentration in patient-1 is considered to reflect existence of recurrence or micrometastasis, although there is no clinical evidence therefor.

### Industrial Applicability

The present invention is applicable to assessment of a degree of progression of prostate cancer and to detection of prostate cancer. Therefore, the present invention is widely applicable to the medical diagnosis fields and the health and medical fields.

All the publications, patens, and patent applications cited herein are incorporated into the present specification as references.

## Claims

1. An analysis method for assessing a degree of progression of prostate cancer, comprising the step of:
measuring an amount of neuropeptide Y in a sample derived from blood or urine obtained from a living organism.

2. The analysis method as set forth in claim 1, further comprising the step of:
measuring an amount of at least one of prostate-specific antigen, mRNA of TMPRSS2:ERG, and PCA3 RNA in a sample derived from blood or urine obtained from the living organism.

3. The analysis method as set forth in claim 1, further comprising the step of:
measuring an amount of prostate-specific antigen in a sample derived from blood or urine obtained from the living organism.

4. The analysis method as set forth in any one of claims 1 through 3, wherein:
the amount of neuropeptide Y is measured by mass spectrometry or luminance assay.

5. The analysis method as set forth in any one of claims 1 through 4, wherein:
the sample is whole blood, a serum, or plasma.

6. The analysis method as set forth in any one of claims 1 through 5, wherein:
in the step of measuring the amount of neuropeptide Y, the sample is subjected to reversed phase extraction twice or more so that a fraction containing neuropeptide Y is obtained, and the amount of neuropeptide Y is measured in the fraction thus obtained.

7. A method for detecting prostate cancer, comprising the step of:
measuring respective amounts of neuropeptide Y and prostate-specific antigen in a sample derived from blood or urine obtained from a living organism.

8. A method for assessing a degree of progression of prostate cancer, comprising the step of:
measuring an amount of neuropeptide Y in a sample derived from blood or urine obtained from a living organism.

9. A test kit for assessing a degree of progression of prostate cancer, comprising:
a peptide probe for measuring an amount of neuropeptide Y in a sample derived from blood or urine obtained from a living organism.

10. The test kit as set forth in claim 9, further comprising:
at least one of (i) an antibody or a peptide probe each for measuring an amount of prostate-specific antigen in a sample derived from blood or urine obtained from the living organism, (ii) a nucleic-acid probe or a nucleic-acid primer each for measuring an amount of mRNA of TMPRSS2:ERG in a sample derived from blood or urine obtained from the living organism, and (iii) a nucleic-acid probe or a nucleic-acid primer each for measuring an amount of PCA3 RNA in a sample derived from blood or urine obtained from the living organism.

11. A test kit for assessing a degree of progression of prostate cancer, comprising:
an antibody for measuring an amount of neuropeptide Y in a sample derived from blood or urine obtained from a living organism; and
at least one of (i) an antibody or a peptide probe each for measuring an amount of prostate-specific antigen in a sample derived from blood or urine obtained from the living organism, (ii) a nucleic-acid probe or a nucleic-acid primer each for measuring an amount of mRNA of TMPRSS2:ERG in a sample derived from blood or urine obtained from the living organism, and (iii) a nucleic-acid probe or a nucleic-acid primer each for measuring an amount of PCA3 RNA in a sample derived from blood or urine obtained from the living organism.

12. A test kit for detecting prostate cancer, comprising:
an antibody or a peptide probe each for measuring an amount of neuropeptide Y in a sample derived from blood or urine obtained from a living organism; and
an antibody or a peptide probe each for measuring an amount of prostate-specific antigen in a sample derived from blood or urine obtained from the living organism.
